# EUROPÄISCHE PATENTANMELDUNG

(11) **EP 1 522 270 A1**
(43) Veröffentlichungstag der Anmeldung: **13.04.2005**
(21) Anmeldenummer: 04023311.6
(22) Anmeldetag: 30.09.2004
(51) Int. Cl.: A61C 8/00, A61L 27/12, A61K 6/00, A61K 6/06, A61L 27/58

(54) **Anordnung zur Rückbildung eines parodontosebedingten Knochendefektes**

(30) Priorität: 10.10.2003 DE 10347232
(71) Anmelder: BEGO Semados GmbH & Co. KG, 28359 Bremen (DE)
(72) Erfinder: Gross, Michael, Dr., 28387 Bremen (DE)
(74) Vertreter: Eisenführ, Speiser & Partner

(57) **Zusammenfassung**

Die Erfindung betrifft eine Anordnung zur Rückbildung eines parodontosebedingten Knochendefekts am Parodontium 2. Die Anordnung weist ein im Bereich des Knochendefekts einbringbares Knochenersatzmaterial 8 und eine um derart eingebrachtes Knochenersatzmaterial anordbare elastische Membran 9 auf zur im wesentlichen vollständigen Abgrenzung des Knochenersatzmaterials 8 von umgebender Gingiva 4. Die Membran weist wenigstens ein Loch 10 zur Durchführung des Zahnes 1 auf.

## Beschreibung

Die Erfindung betrifft eine Anordnung zur Rückbildung eines parodontosebedingten Knochendefektes an Parodontium.

Bekannt sind verschiedene Maßnahmen zur Behandlung von Parodontose. Jedoch haben diese Maßnahmen gemein, dass sie auf den Erhalt des Status Quo gerichtet sind. Das heißt, ein gezielter Knochenaufbau im Bereich des parodontosegeschädigten Parodontiums findet nicht statt. Dies liegt daran, dass keine geeigneten, einfach durchzuführenden Maßnahmen zum Knochenaufbau zur Verfügung stehen.

Der Erfindung liegt daher das Problem zugrunde, Mittel für eine verbesserte Parodontosebehandlung zur Verfügung zu stellen.

Die Erfindung löst dieses Problem mit einer Anordnung zur Rückbildung eines parodontosebedingten Knochendefektes am Parodontium, die aufweist:
- ein im Bereich des Knochendefekts einbringbares Knochenersatzmaterial und
- eine um eingebrachtes Knochenersatzmaterial anordbare elastische Membran zur im wesentlichen vollständigen Abgrenzung des Knochenersatzmaterials von umgebender Gingiva,
- wobei die Membran wenigstens ein Loch zur Durchführung eines Zahnes aufweist.

Der Erfindung liegt die Erkenntnis zugrunde, dass durch eine elastische Membran - die derart über einen Zahn stülpbar ist, dass die Membran dicht am Zahn abschließt und dass die Membran ein im Bereich des Knochendefektes-eingebrachtes Knochenersatzmaterial im wesentlichen vollständig gegenüber der umgebenden Gingiva abdeckt - eine schnelle und einfache und daher auch unter Kostengesichtspunkten durchführbare Behandlungsmethode zur Rückbildung eines Knochendefektes bereitgestellt werden kann. Auf diese Weise wird parodontosegeschädigter Knochen nicht nur erhalten, sondern wieder aufgebaut.

Vorteilhafterweise weist das Loch der Membran eine Querschnittsfläche auf, die vor der Durchführung des Zahnes durch das Loch bzw. der Überstülpung der Membran über den Zahn kleiner ist als eine Querschnittsfläche des entsprechenden Zahnes, bei der die Membran ihre Endlage erreicht. Hierdurch wird sichergestellt, dass die Membran dicht den Zahn umgibt und ein Eindringen von Verunreinigungen in den Bereich der Knochenregeneration vermieden wird.

Vorzugsweise ist die Membran vom menschlichen oder tierischen Körper resorbierbar. Somit entfällt die Notwendigkeit der späteren operativen Entfernung der Membran.

Besonders bevorzugt ist die Membran semipermeabel. Vorteilhafterweise ist eine semipermeable Membran derart in der Anordnung ausgerichtet, dass ein Flüssigkeitsaustritt aus dem Bereich des augmentierten Knochendeffekts möglich ist bzw. aus dem knochennahen Teil Flüssigkeit in das umliegende Gewebe abfließen kann. Auf diese Weise kann sichergestellt werden, dass seröse Flüssigkeit, insbesondere Wundwasser, aus dem Bereich des Knochendeffektes in das umliegende Gewebe abfließen kann. Insbesondere ist die Membran dabei derart ausgebildet, dass zelluläre Komponenten weder von der einen noch von der anderen Seite der Membran durch die Membran hindurchtreten können.

Alternativ ist eine semipermeable Membran umgekehrt in der Anordnung ausgerichtet, das heißt derart, dass Flüssigkeiten vom umliegenden Gewebe in den Bereich des Knochendeffektes eindringen kann. Eine derartige Anordnung ist dann vorteilhaft, wenn der Bereich des Knochendefektes und damit das eingebrachte Knochenersatzmaterial vom umliegenden Gewebe mit Nährstoffen, insbesondere für eine Beschleunigung des Knochenaufbaus versorgt werden soll.

Das eingesetzte Knochenersatzmaterial ist vorteilhafterweise osteoinduktiv. Osteoinduktive Materialien induzieren eine Knochenbildung - auch ohne Kontakt zum verbliebenen Knochen. Die Verwendung von osteoinduktivem Material ist deshalb besonders vorteilhaft, da es eine schnelle Knochenbildung begünstigt.

Vorteilhafterweise ist das Knochenersatzmaterial synthetisch (hergestellt). Es kommt somit ohne organische Produkte bzw. Substanzen aus, die Krankheiten übertragen könnten.

Das Knochenersatzmaterial ist vorzugsweise als Granulat ausgebildet. Vorteilhafterweise sind die Granulatkörnchen mit Blut vermischt, um eine klebrige Masse zu bilden, die im Bereich des Knochendefekts aufgetragen werden kann.

Bei einer anderen Ausführungsform ist das Knochenersatzmaterial in einem Gel eingebettet. Auf diese Weise kann es leicht auf den Knochendefekt aufgebracht werden, ohne wieder abzufallen.

Besonders bevorzugt weist die Anordnung Befestigungsmittel zum Befestigen der Membran am Knochen auf. Hierdurch wird eine Fixierung und Abdichtung der Membran im Bereich des Knochens erreicht. Auf diese Weise ist sichergestellt, dass auch in den Randbereichen der Membran kein Kontakt zwischen dem Knochenersatzmaterial und der umgebenden Gingiva entsteht.

Vorzugsweise sind die Befestigungsmittel ebenfalls vom Körper resorbierbar.

Weitere vorteilhafte Ausführungsformen der Erfindung ergeben sich aus den Unteransprüchen sowie aus den anhand der beigefügten Zeichnung näher erläuterten Ausführungsbeispiele. In der Zeichnung zeigt:
- Fig. 1: eine seitliche Schnittansicht durch einen Zahn in einem gesunden Parodontium;
- Fig. 2: eine seitliche Schnittansicht durch einen Zahn in einem parodontosegeschädigten Parodontium, bei dem sich die Gingiva sowie der darunter liegende Knochen zurückentwickelt hat;
- Fig. 3: eine seitliche Schnittansicht des Zahns mit parodontosegeschädigtem Parodontium gemäß Fig. 2 mit teilweise abgetrennter Gingiva;
- Fig. 4: eine seitliche Schnittansicht des Zahns mit parodontosegeschädigten Parodontium mit teilweise abgetrennter Gingiva gemäß Fig. 3 und im Bereich eines Knochendefekts applizierten Knochenersatzmaterials;
- Fig. 5: eine seitliche Schnittansicht des Zahns und Parodontiums gemäß Fig. 4 mit über dem applizierten Knochenersatzmaterial angebrachter Membran;
- Fig. 6: eine seitliche Schnittansicht des Zahns und Parodontiums mit appliziertem Knochenersatzmaterial und abdeckender Membran gemäß Fig. 5, wobei die Gingiva über die Membran gelegt ist;
- Fig. 7: eine seitliche Schnittansicht des Zahns und Parodontiums gemäß Fig. 6 mit reorganisiertem Knochendefekt und teilweise resorbierter Membran und resorbiertem Befestigungsmittel;
- Fig. 8: eine seitliche Schnittansicht des Zahns und Parodontiums gemäß Fig. 7 mit vollständig resorbierter Membran und vollständig resorbiertem Befestigungsmittel; und
- Fig. 9: eine Ansicht von oben einer Membran zur Verwendung in einer erfindungsgemäßen Anordnung.

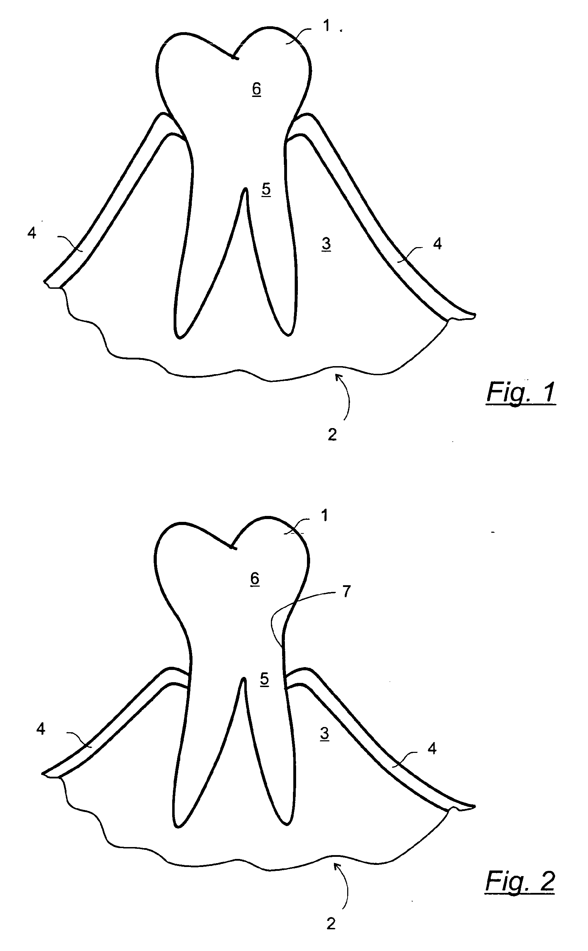

Fig. 1 zeigt einen Zahn 1, der in einem gesunden Parodontium 2 eingewachsen ist. Das Parodontium umfasst (u.a.) einen knöchernen Abschnitt 3, der von einer Gingiva 4 bedeckt ist. Bei diesem gesunden Parodontium ist die bzw. sind die Wurzeln 5 des Zahnes 1 vollständig eingewachsen und es ragt lediglich die Zahnkrone 6 aus der Gingiva heraus.

Fig. 2 zeigt einen an Parodontose erkrankten Zahn, bei dem sich der Knochen 3 im Bereich des Zahnhalses 7 zurückgebildet hat. Da die den Knochen 3 bedeckende Gingiva 4 mit dem Knochen 3 verwachsen ist, bildet sich auch die Gingiva 4 zurück. Das Parodontium 2 ist geschädigt. Der Zahn 1 lockert sich. Im vorgeschrittenen Stadium kommt es schließlich zum Zahnausfall.

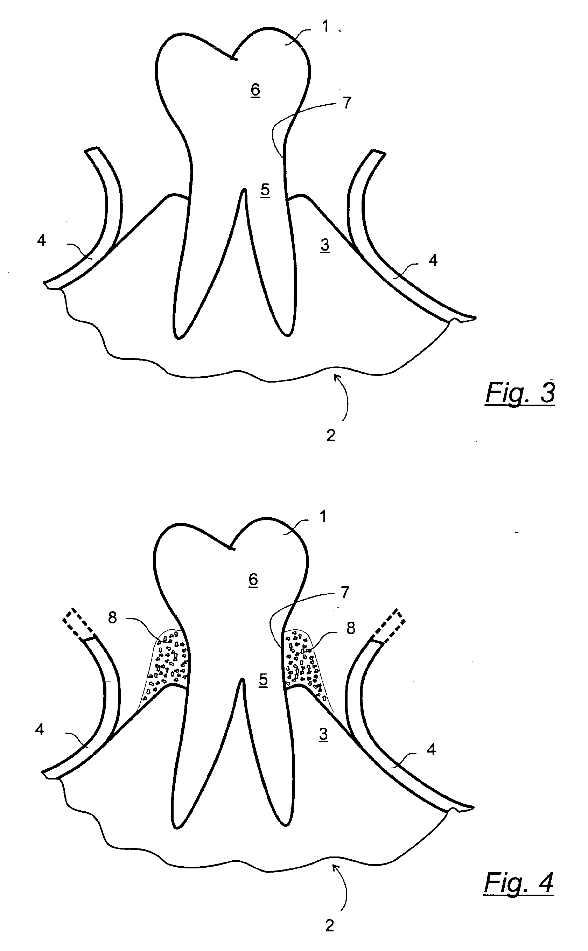

Fig. 3 bis 8 zeigen ein Ausführungsbeispiel einer erfindungsgemäßen Maßnahme zur Behandlung derartiger Parodontose. Wie in Fig. 3 gezeigt, wird zunächst die Gingiva 4, ausgehend vom Zahnhals 7, vom Knochen und Zahn 3 gelöst, beispielsweise mittels Messerschnitten abgetrennt. Auf diese Weise wird dem Operateur Platz geschaffen, um anschließend weitere Maßnahmen am Knochen 3 im Bereich des Knochendefektes und entlang des Zahnhalses bzw. der Wurzeloberfläche 7 durchzuführen.

Fig. 4 zeigt zwei dieser Maßnahmen. Bei einer ersten Maßnahme wird entlang des Zahnhalses 7 ein Knochenersatzmaterial 8 aufgetragen, um den infolge der Parodontoseerkrankung verloren gegangenen Knochenabschnitt im Bereich des Zahnhalses 7 wieder herzustellen. Bei diesem Knochenersatzmaterial handelt es sich vorzugsweise um ein osteoinduktives Knochenersatzmaterial, wie es beispielsweise in US 6 511 510 B1 beschrieben ist. Das Knochenersatzmaterial ist synthetisch hergestellt, das heißt es weist insbesondere keine organischen Produkte auf. Das Knochenersatzmaterial weist vielmehr keramisches Material, insbesondere Kalziumphosphat, wie Hydroxylapatit, mit einem Anteil von ca. 70% bis 85%, bspw. im wesentlichen 75% des Knochenersatzmaterials auf. Ferner weist das Knochenersatzmaterial 15% bis 30% β-Trikalziumphosphate auf.

Dieses Knochenersatzmaterial weist femer Mikroporen und Makroporen auf. Durch diese Poren ergibt sich eine Gesamtporosität des Materials von 70% bis 80%. Die Mikroporen haben eine Größe von ca. 0,05µm bis 20µm und die Makroporen eine Größe von 0,1mm bis 1,5mm.

Das Knochenersatzmaterial ist als Granulat ausgebildet, wobei die Granulatkörnchen eine Größe von ca. 0,25mm bis 3,00mm, bevorzugt 0,5mm bis 2,00mm, aufweisen.

Dieses Knochenersatzmaterial weist osteoinduktive Eigenschaften auf. Das heißt, es ist knochen bildend und zwar auch ohne dass ein Kontakt zum verbliebenen Knochen notwendig wäre. Im Gegensatz dazu unterstützen osteokonduktive Materialien zwar den Knochenaufbau. Jedoch findet ein Knochenaufbau nur dann statt, wenn das Material Kontakt zu verbliebenen bzw. bereits nachgewachsenem Knochen hat.

Osteoinduktives Knochenersatzmaterial ist besonders vorteilhaft, da es einen schnellen Knochenaufbau, insbesondere einen schnellen vertikalen Knochenaufbau ermöglicht, weil eine Knochenbildung in vielen Bereichen innerhalb des aufgebrachten Knochenersatzmaterials einsetzt.

Vorteilhafterweise wird das als Granulat vorliegende Knochenersatzmaterial mit Blut, Blutpräparaten, z.B. Platelet Rich Plasma, und/oder Natriumchloridlösung (NaCl/physiologische Kochsalzlösung) vermischt, um eine streichfähige, modellierbare Masse zu erhalten. Erst eine derartige Masse lässt sich in der gewünschten Form im Bereich des Knochendefektes um den Zahnhals 7 herum auf den verbliebenen Knochen 3 auftragen. Ein rein als Granulat vorliegendes Knochenersatzmaterial würde während der Operation nach unten an ungewünschte Stellen fallen.

Alternativ oder zusätzlich wird das Knochenersatzmaterial in ein Gel eingebettet. Dieses Gel ist derart ausgebildet, dass es ebenfalls plastisch formbar und modellierbar im Bereich des Knochendefektes aufbringbar ist.

Die zweite in Fig. 4 gezeigte Maßnahme ist eine Verlängerung der Gingiva 4. Diese ist erforderlich, um die Gingiva 4 auch über das am Zahnhals 7 aufgebrachte Knochenersatzmaterial 8 wieder derart auflegen zu können, dass das Knochenersatzmaterial 8 vollständig abgedeckt wird, das heißt dass die Gingiva 4 wieder bis an die Zahnkrone 6 heranreicht. Hierzu stehen bekannte Verfahren zur Verfügung, wie z.B. die Periostschlitzung.

Nach der Präparation eines Mukoperiostlappens, also der Ablösung von Zahnfleisch und Knochenhaut vom Knochen, wird die nicht dehnbare Knochenhaut mit einem Skalpell vorsichtig durchtrennt, ohne weitere Weichgewebe zu verletzen. Nach der Durchtrennung lässt sich das Gewebe (Mukosa/Gingiva) dehnen und mobilisieren, wodurch die gewünschte Verlängerung eintritt.

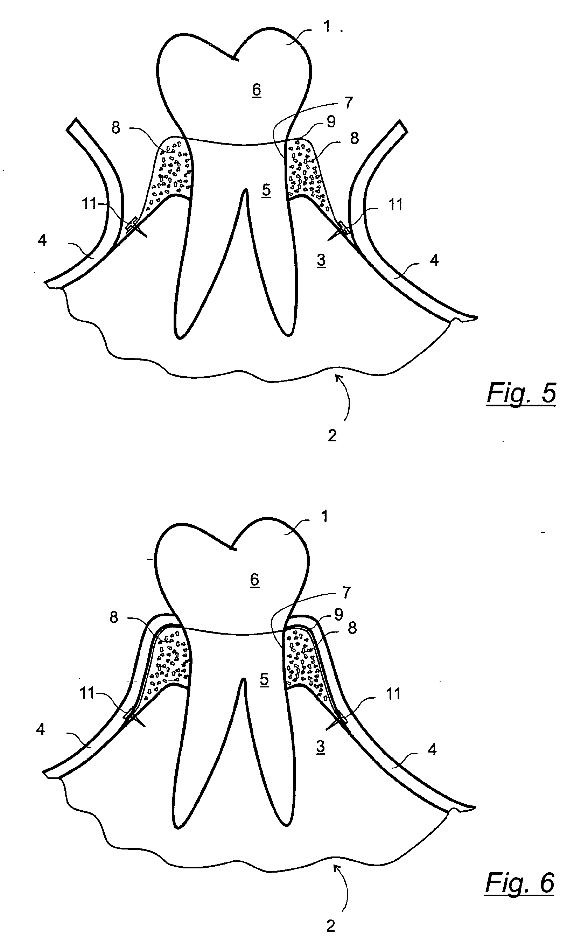

Wie in Fig. 5 gezeigt, wird anschließend über das entlang des Zahnhalses 7 auf dem verbliebenen Knochen 3 aufgebrachte Knochenersatzmaterial 8 eine gummielastische, implantierbare, insbesondere gewebeverträgliche Membran 9 angebracht, die auch wenigstens teilweise um den Zahn 1 und den Knochen 3 herum angeordnet ist. Diese Membran 9 hält zum einen das aufgetragene Knochenersatzmaterial 8 an seiner Position. Zum anderen verhindert diese Membran 9 jedoch auch eine Einsprossung von Epithel über die Gingiva 4 in den späteren knöchernen Abschnitt, der in diesem Stadium noch aus Knochenersatzmaterial 8 besteht.

Die Membran 9 besteht vorzugsweise aus einem resorbierbaren Material. Deshalb muss die Membran später nicht wieder entfernt werden, was eine erneute Entfernung der Gingiva 4 erforderlich machen würde. Vielmehr löst der Körper die Membran 9 selbständig auf.

Die Membran ist derart ausgebildet, dass sich eine nahtfreie Abdichtung um den Zahn 1 herum ergibt. Insbesondere ist die Membran aus einem reißfesten Material gebildet.

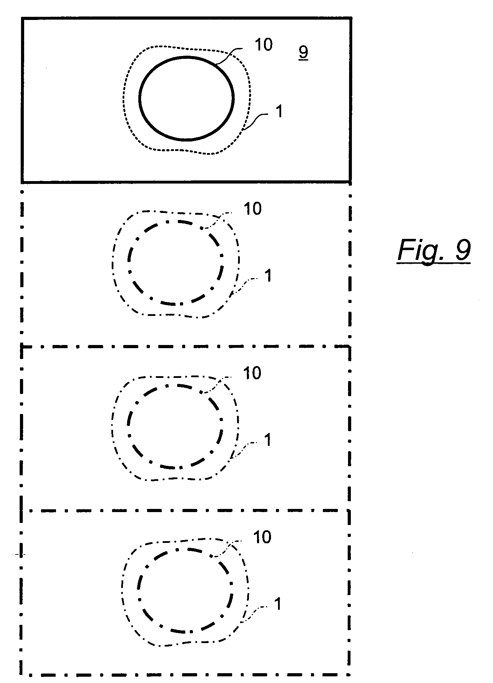

Fig. 9 zeigt eine mögliche Ausbildung der Membran 9. Die Membran 9 weist ein oder mehrere Löcher 10 zum Durchführen eines oder mehrerer Zähne 1 auf. Hierdurch wird ein Überstülpen der Membran über einen Zahn 1 ermöglicht. Um eine gute Abdichtung der Membran 9 entlang des Zahnes 1 zu erhalten, ist die durch den Rand des Lochs 10 umschriebene Querschnittsfläche kleiner als die von der Kontur des Zahns 1 umschriebene Querschnittsfläche im Bereich der Endlage der Membran 9.

Wie in Fig. 9 durch strichpunktierte Linien dargestellt, kann die Membran mehrere Löcher vorsehen. Dies ist insbesondere dann vorteilhaft, wenn nicht nur ein einzelner Zahn einer Parodontosebehandlung unterzogen wird, sondern mehrere Zähne zu behandeln sind. Die Löcher 10 sind dabei derart angeordnet, dass sie nicht miteinander in Berührung treten, sondern dass jeweils zwischen zwei benachbarten Löchern 10 ein Abschnitt der Membran verbleibt. Hierdurch wird eine sichere Abdichtung der Membran um jeden einzelnen Zahn gewährleistet.

Wie in Fig. 5 gezeigt, wird nach dem Überstülpen der Membran 9 über einen Zahn 1 die Membran am Knochen 3 mittels geeigneter Befestigungsmittel 11, bspw. nach Art eines Pins, einer Heftzwecke, Klammer oder Schraube oder mittels eines Kleber befestigt. Derartige Befestigungsmittel werden entweder direkt in den Knochen 3 eingebracht oder, soweit möglich, derart angeordnet, dass sie den unteren Bereich des eingebrachten Knochenersatzmaterials 8 vollständig umschließen bzw. umklammern und die Membran 9 gegen den Knochen 3 drücken.

Die Befestigungsmittel 11 sind vorzugsweise ebenfalls aus einem resorbierbaren Material.

Wie in Fig. 6 gezeigt, wird anschließend über die Anordnung aus Knochenersatzmaterial 8, Membran 9 und Befestigungsmittel 11 die Gingiva 4 gelegt und entlang der anfänglich vorgenommenen Schnitte vernäht.

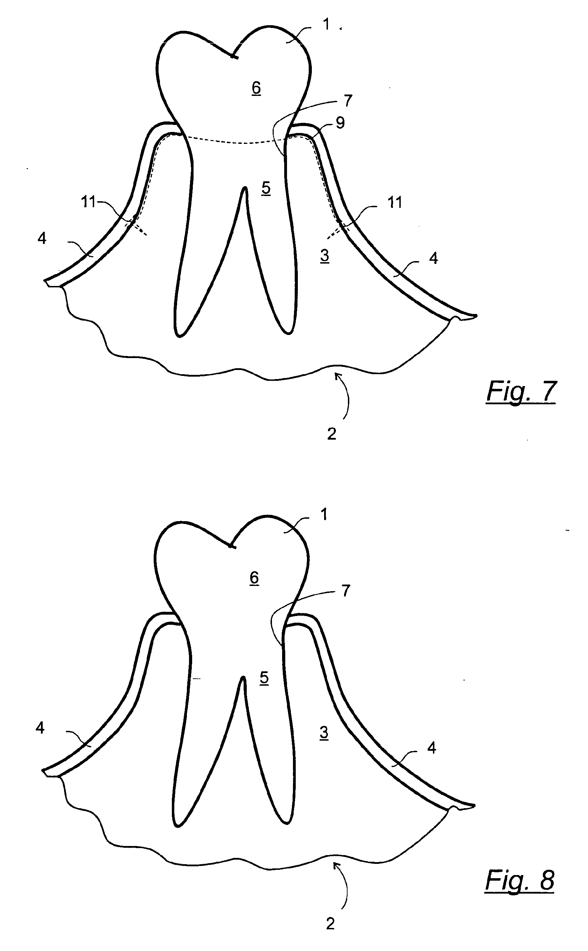

Wie in Fig. 7 gezeigt, wird das Knochenersatzmaterial vollständig in Knochen umgewandelt, wobei sich auch zwischen den Knochenersatzmaterialkörnchen neuer Knochen bildet. Auf diese Weise entsteht wieder ein natürlicher Knochen im Bereich des ursprünglichen, parodontosebedingten Knochendefekts. In diesem Stadium des bereits wiederaufgebauten Knochens lösen sich die Membran 9 und die Befestigungsmittel 11 auf.

Wie in Fig. 8 gezeigt, ist am Ende des Umbauprozesses als Knochenersatzmaterial der Zahn 1 wieder bis zur Zahnkrone 6 vom Parodontium 2 umwachsen. Insbesondere ist der Zahnhals 7 wieder von Knochen 3 umgeben. Der Zustand von Fig. 8 entspricht daher im wesentlichen dem Zustand von Fig. 1, so dass der parodontosebedingte Knochendefekt vollständig rückgebildet werden konnte.

Vorteilhafterweise wird zu Beginn der Maßnahmen eine Schiene an den Zähnen 1 angebracht, um deren Mobilität einzuschränken, das heißt die Zähne 1 werden immobilisiert. Die Schienung erfolgt bspw. durch Befestigen eines Drahtes oder eines Gitters an den Zähnen bzw. durch eine mit Draht und/oder Gitter verstärkte Kunststoffschiene, die an den Zähnen 1 befestigt wird. Alternativ werden gegossene Metallschienen verwendet, die mit den Zähnen verklebt werden können. Eine weitere Alternative besteht in der Verwendung einer individuell (Laser)gesinterten, gefrästen, gegossenen oder geschmolzenen Kunststoff- und/oder Metall-Schiene.

Dank der erfindungsgemäßen Anordnung aus, insbesondere osteoinduktivem, Knochenersatzmaterial und einer das Knochenersatzmaterial umgebenden elastischen Membran können Knochendefekte infolge von Parodontose vollständig rückgebildet werden. Insbesondere erreicht man eine Knochenregeneration bis zur ursprünglichen Höhe. Die Zähne befinden sich wieder in einem stabilen Parodontium. Zahnlockerungen wurden beseitigt.

## Patentansprüche

1. Anordnung zur Rückbildung eines parodontosebedingten Knochendefektes am Parodontium (2), die aufweist:
- ein im Bereich des Knochendefekts einbringbares Knochenersatzmaterial (8) und
- eine um eingebrachtes Knochenersatzmaterial (8) anordbare elastische Membran (9) zur im wesentlichen vollständigen Abgrenzung des Knochenersatzmaterials (8) von umgebender Gingiva (4),
- wobei die Membran (9) wenigstens ein Loch (10) zur Durchführung eines Zahnes (1) aufweist.

2. Anordnung nach Anspruch 1, wobei-die Membran (9) vom menschlichen oder tierischen Körper resorbierbar ist.

3. Anordnung nach Anspruch 1 oder 2, wobei die Membran (9) semipermeabel ist.

4. Anordnung nach einem der vorhergehenden Ansprüche, wobei die Membran (9) reißfest und elastisch dehnbar ist.

5. Anordnung nach einem der vorhergehenden Ansprüche, wobei das Loch (10) eine Querschnittsfläche aufweist, die vor der Durchführung eines Zahnes (1) kleiner ist als eine Querschnittsfläche des durchzuführenden Zahnes (1 ).

6. Anordnung nach einem der vorhergehenden Ansprüche, wobei das Knochenersatzmaterial (8) osteoinduktiv ist.

7. Anordnung nach einem der vorhergehenden Ansprüche, wobei das Knochenersatzmaterial (8) synthetisch bzw. synthetisch hergestellt ist.

8. Anordnung nach einem der vorhergehenden Ansprüche, wobei das Knochenersatzmaterial (8) keramisches Material, insbesondere Kalziumphosphat, insbesondere Hydroxylapatit, aufweist.

9. Anordnung nach einem der vorhergehenden Ansprüche, wobei das Knochenersatzmaterial (8) 70% bis 85% Hydroxylapatit aufweist.

10. Anordnung nach einem der vorhergehenden Ansprüche, wobei das Knochenersatzmaterial (8) 15% bis 30% β-Trikalziumphosphate aufweist.

11. Anordnung nach einem der vorhergehenden Ansprüche, wobei das Knochenersatzmaterial (8) Mikroporen und Makroporen sowie eine Gesamtporosität von 70% bis 80% aufweist, wobei die Mikroporen eine Größe von 0,05µm bis 20µm und die Makroporen eine Größe von 0,1mm bis 1,5mm aufweisen.

12. Anordnung nach einem der vorhergehenden Ansprüche, wobei das Knochenersatzmaterial (8) als Granulat ausgebildet ist.

13. Anordnung nach Anspruch 12, wobei die Granulatkörnchen eine Größe von 0,25mm bis 3,00mm, bevorzugt 0,5mm bis 2,00mm, aufweisen.

14. Anordnung nach einem der vorhergehenden Ansprüche, wobei das Knochenersatzmaterial (8) mit Blut, Blutpräparaten und/oder Natriumchloridlösung vermischt ist.

15. Anordnung nach einem der vorhergehenden Ansprüche, wobei das Knochenersatzmaterial (8) in ein Gel eingebettet ist.

16. Anordnung nach einem der vorhergehenden Ansprüche, die ferner Befestigungsmittel (11) zum Befestigen der Membran (9) am Knochen (3) aufweist.

17. Anordnung nach Anspruch 16, wobei die Befestigungsmittel (11) nach Art eines Pins, einer Heftzwecke und/oder einer Klammer oder Kleber ausgebildet sind.

18. Anordnung nach Anspruch 16 oder 17, wobei die Befestigungsmittel (11) vom menschlichen oder tierischen Körper resorbierbar sind.

19. Anordnung nach einem der vorhergehenden Ansprüche, die ferner eine Schiene zur Immobilisierung lockerer Zähne (1) aufweist.
